# EUROPEAN PATENT APPLICATION

(11) **EP 1 010 974 A1**
(43) Date of publication of application: **21.06.2000**
(21) Application number: 98123846.2
(22) Date of filing: 15.12.1998
(51) Int. Cl.: G01N 1/12, G01N 33/18, E21B 49/08

(54) **Sampling probe for the in-situ extraction of organic micropollutants**

(71) Applicant: Euratom, 2920 Luxembourg (LU)
(72) Inventor: Bo, Larsen, Dr., 21024 Barzola (IT); Facchetti, Sergio, Dr., 21100 Varese (IT); Franchini, Paolo, Dr., 40133 Bologna (IT); Montanarella, Luca, Dr., 21026 Gavirate (IT)
(74) Representative: Strych, Werner Maximilian Josef, Dr.

(57) **Abstract**

A sampling probe for the in situ extraction of organic micropollutants from groundwater the object of which is to enable the application of the known solid-phase micro-extraction method to groundwater analysis is described. For achieving this aim it is proposed to provide a hollow elongated probe housing having a chamber and an extraction fiber being coated with an adsorbent material for adsorbing an analyte, said extraction fiber being arranged in said chamber, said probe housing having at least one opening allowing said groundwater to enter said chamber in such a manner that said extraction fiber immerses at least partially into said groundwater, and to provide a connector means for connecting a pulling means for lowering and retracting said sampling probe into and from a well. Alternatively it is proposed to arrange said extraction fiber movably for movement in an axial direction of said probe housing between an initial position in which said extraction fiber is located in said chamber and an exposed position in which said extraction fiber is exposed to said groundwater outside said chamber, and to provide a mounting means for mounting said sampling probe in the head of a drilling rod for drilling a well.

## Description

The present invention refers to a sampling probe for the in situ extraction of organic micropollutants from groundwater.

The ability to analyze organics in water matrix samples in the shortest period of time and in the most cost efficient manner is a key concern to any environmental analytical chemist. One of the major costs of groundwater investigations and cleanup is chemical analysis. Currently, the mehtod of liquid-liquid extraction is the most popular form of analysis of such samples. However, this procedure is time consuming and very expensive requiring high purity solvents. In addition, this method is labour intensive and very difficult to automate.

Even more problematic is the analysis of groundwater contaminants due to obvious difficulties in sample collection. This is particularly true for the analysis of volatile organic compounds (VOC's), which get easily lost during pumping of the sample to the surface.

Traditional drilling and sampling technologies often utilize a method whereby the drilling proceeds until the drill behaviour and examination of cuttings indicate that more sophisticated sampling is required. At that point, the drill rod and bit are withdrawn from the hole, a sampler, such as a split spoon sampler, is sent down the hole on the end of a drive tube, the sampler is driven into the bottom of the hole, and the sample is withdrawn.

According to Larsen B.; Montanarella L. and Mousty F.; "Identification and Characterisation of Organic and Inorganic Contaminants, Sample Cleanup and Analytical Methods", in A. Avogadro and R.C. Ragaini (eds.), Technologies for Environmental Cleanup: Soil and Groundwater, 61-114, 1993, ECSC; EEC; EAEC; Brussels and Luxembourg well established methods like Purge&Trap, Head-Space and Liquid-Liquid extraction are available for the analysis of groundwater samples from groundwater wells. Unfortunately, those methods require pumping of the groundwater to the surface, sampling into appropriate containers and transport to the analytical laboratory. During all these steps serious sample losses and sample composition variations are possible and have been already observed for a range of pollutants.

Further, the technique of solid-phase micro-extraction (SPME) is known in the prior art. Said technique is based on the introduction of a fused silica fiber coated with an adsorbent material directly into the sample followed by adsorption of the analyte to the fiber coating and subsequent thermal desorption directly into a gas chromatograph (GC) without any clean-up or pre-concentration. This method is a less expensive and faster alternative to the well-established methods of Purge&Trap, Head-Space and Liquid-Liquid extraction.

The SPME-method is ideally suited to field sampling because it is portable and independent of instrument configuration. It is impossible to plug the fiber with particulate matter and the technique is independent of the sample volume. This technique has been already proposed for the in situ measurement of surface waters like rivers and lakes.

However, the prior art does not provide a sampling probe which enables the application of the SPME-method to the investigation of groundwaters.

Accordingliy, it is the object of the present invention to provide a sampling probe for the in situ extraction of organic micropollutants which enables the analyst to apply the known SPME-method to groundwater in an easy and accurate manner.

This object is achieved by a sampling probe for the in situ extraction of organic micropollutants from groundwater having the features of claim 1 or 7. Further features of the present invention are subject to the subclaims.

According to a first embodiment of the present invention the sampling probe for the in situ extraction of organic micropollutants from groundwater has a hollow elongated probe housing having a chamber and an extraction fiber being coated with an adsorbent material for adsorbing an analyte, in particular the specific groundwater to be investigated. The extraction fiber is arranged in the chamber and the probe housing has at least one opening allowing the analyte to enter the chamber in such a manner that said extraction fiber immerses at least partially into said analyte. Finally, a connector means for connecting a pulling means for lowering and retracting the sample probe into and from a well is provided.

According to a second embodiment of the present invention the sampling probe for the in situ extraction of organic micropollutants from groundwater has a hollow elongated probe housing having a chamber and an extraction fiber being coated with an adsorbent material for adsorbing an analyte. In contrast to the first embodiment the extraction fiber of the second embodiment is a movably arranged for movement in an axial direction of the probe housing between an initial or retracted position in which the extraction fiber is located in the chamber and an exposed position in which the extraction fiber is exposed to the analyte outside the chamber. Further, the second embodiment has mounting means for mounting the sampling probe in the head of a drilling rod for drilling a well. Thus, the second embodiment offers in particular the advantage that it is not necessary to retract the drilling rod from the well before the coated extraction fiber may be brought into contact with the analyte.

The sampling probes according to the present invention have a simple and rugged design enabling the analyst to conduct a rapid on-site screening of groundwater contaminants. Further, the oparation of the sampling probes is very easy and a true groundwater contamination measurement is feasible. Last but not least the present invention leads to dramatic cost reduction compared to traditional methods applied in the prior art for analysing groundwaters.

Next, two examplary embodiments of the present invention are described under reference to the attached drawings.
- Fig. 1: shows a cross sectional view of a first embodiment of the present invention and
- Fig. 2: shows a cross sectional view of a second embodiment of the present invention.

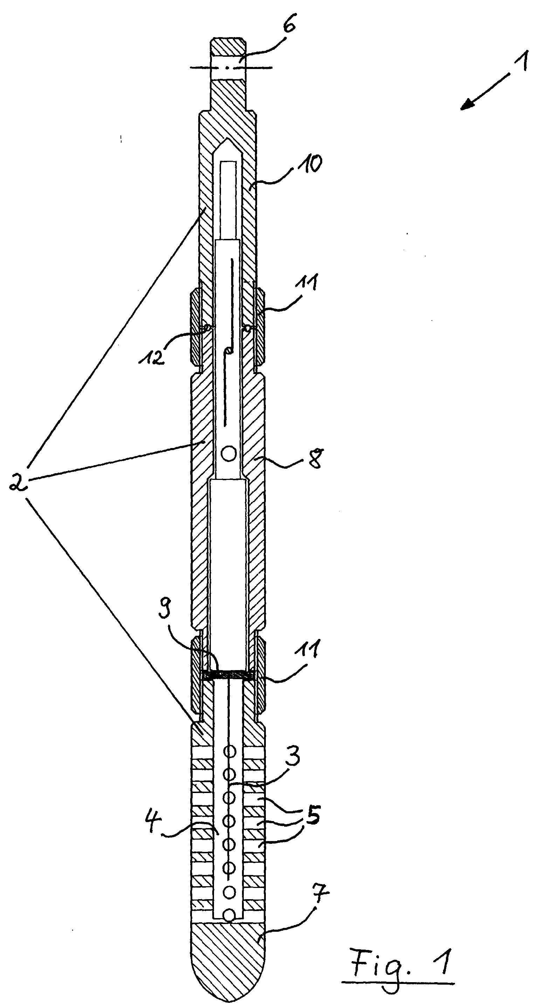

In Fig. 1 a sampling probe 1 of the first embodiment is shown. Sampling probe 1 has a hollow and substantially cylindrical probe housing 2 which is formed by three components, namely a probe head portion 7, a head holder portion 8 and an end cap portion 10. The probe housing 2, i.e. the probe head portion 7, the head holder portion 8 and the end cap portion 10, is preferably made of a metallic material. Other suitable materials can be used alternatively. Further, the probe head portion 7 is threadedly connected to the head holder portion 8 while the head holder portion 8 in turn is threadedly connected with the end cap portion 10. For these connections respective sleeves 11 having an inside thread corresponding to the respective outside thread of probe head portion 7, head holder portion 8 and end cap portion 10 are provided. As can be seen in Fig. 1, a sealing means, preferably a sealing ring 12, is located between head holder portion 8 and end cap portion 10.

At the upper end of the end cap portion 10 a connector means in the form of a throughhole 6 is illustrated. As can be further seen in Fig. 1 the upper end portion of end cap portion 10 is smoothed down. Throughhole 6 serves for connecting sample probe 1 with a pulling means, preferably a cord, a wire cable, a chain or the like, for lowering and retracting sampling probe 1 into and from the groundwater well.

Although sampling probe 1 is shown in Fig. 1 as being a three piece probe it is understood that sampling probe 1 may also be made from two or even one piece only.

At the lower end of sampling probe 1 the probe head portion 7 including an elongated chamber 4 for receiving a coated extraction fiber 3 in its interior is illustrated. Chamber 4 was made by drilling a pocket hole into probe head portion 7. As can be seen, chamber 4 extends along approximately three quarter of the overall length of probe head portion 7. The leading end of probe head portion 7 is rounded.

In order to bring the groundwater to be investigated in contact with the coating of the extraction fiber 3 a plurality of openings in the form of radial bores 5 is provided in probe head portion 7. These bores 5 are connecting chamber 4 with the surrounding of probe head portion 7, thus enabling a rapid exchange of the groundwater surrounding immersed probe head portion 7 with the air in chamber 4. As can be seen in Fig. 1, bores 5 are distributed along the longitudinal axis of probe head portion 7 as well as in its circumferential direction. In the illustrated embodiment bores 5 are laying in two planes intersecting each other in a right angle. During the process of immersing the probe head portion 7 into the groundwater the bores 5 being above the hight of still groundwater are serving as venting apertures for venting the air from chamber 4 which is displaced by the groundwater entering chamber 4 through lower bores 5.

Alternatively, instead of bores 5 at least one elongated slot extending substantially parallel to the longitudinal axis of probe head portion 7 might be provided. Such a slot or purality of slots would have the same venting function as described above in connection with bores 5.

According to Fig. 1 coated extraction fiber 3 is fixed in a fiber holder means which is a circular holder plate 9 in the shown embodiment. The outer diameter of holder plate 9 is larger than both the inner diameter of chamber 4 and the inner diameter of interior cavity of head holder portion 8. Accordingly, holder plate 9 can be clamped between the lower front face of head holder portion 8 and the upper front face of probe head portion 7. Thus, coated extraction fibre 3 is stationary relative to all other components of sampling probe 1.

Prior to use of sampling probe 1 first of all a groundwater well is drilled by means of conventional drilling rod equipment. If the well has the desired depth the drilling rod is retracted from the well and the analyst lowers sampling probe 1 down into the well with the help of a pulling means connected to throughhole 6. The process of lowering is continued until extraction fiber 3 extending along approximately two third of the length of probe head portion 7 immerses into the groundwater entering chamber 4 through bores 5. Then, the SPME-process takes place and fiber coating of extraction fiber 3 adsorbes a sample of the groundwater. After an appropriate equilibration time, i.e. 30 minutes to several hours, sampling probe 1 is retracted to the surface by pulling the pulling means and extraction fibrer 3 can be immediately desorbed in a portable gas chromatograph or in a mobile laboratory.

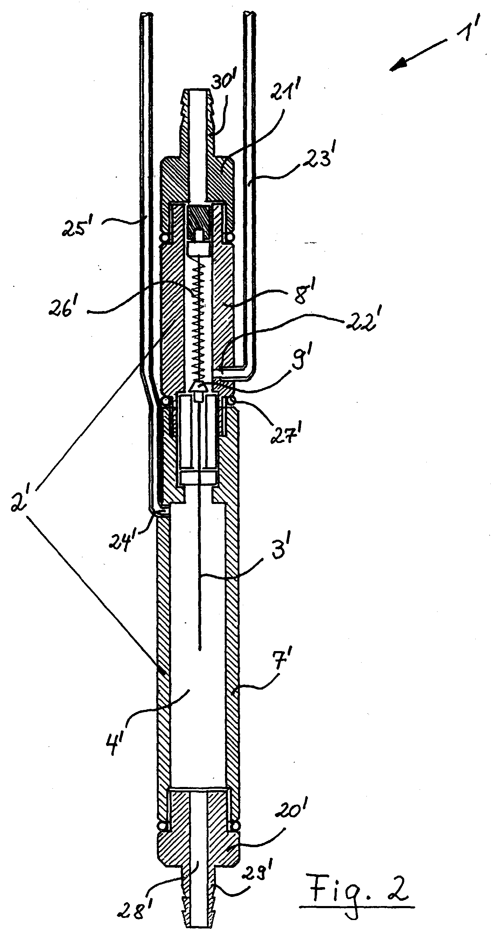

Fig. 2 shows a sampling probe 1' according to the second embodiment of the present invention. Sampling probe 1' is adapted to be placed inside the head of a drilling rod used for drilling the groundwater well.

Sampling probe 1' has a probe housing 2' being formed by two components, namely a lower probe portion 7' and an upper probe portion 8'. Lower probe portion 7' and upper probe portion 8' are threadedly connected and a sealing ring 27 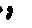 is located between their front faces laying opposite each other. Lower probe portion 7' and upper probe portion 8' are preferably made of a metallic material. However, other suitable materials can be used alternatively. As can be seen in Fig. 2, an elongated chamber 4' is provided in lower probe portion 7'. Chamber 4' receives coated extraction fiber 3' which is, in contrast to the first embodiment, movable relative to probe housing 2' and in particular to lower probe portion 7'.

In Fig. 2 extraction fiber 3' is shown in its initial position in which it is located in chamber 4'. For bringing extraction fiber 3' into contact with groundwater it is necessary to move it into an exposed position in which it is exposed to the groundwater outside or below chamber 4'. In regular operation of sampling probe 1' no groundwater will enter chamber 4'.

Extraction fiber 3' is fixed on a fiber holder means 9' which is mushroom-shaped in the shown embodiment. For moving extraction fiber 3' downward it is necessary to move fiber holder means 9' in the downward dirction. For this purpose upper probe portion 8' has a first port 22' for connecting a pressure line 23' connectable to a pneumatic source at the surface. Preferably, the pneumatic source delivers compressed air for actuating the downward movement of fiber holder means 9'. The compressed air passing through pressure line 23' enters the interior cavity in upper probe portion 8' at a point above fiber holder means 9' which is in its initial position. Then, mushroom-shaped fiber holder means 9' acts comparable to a differential piston. The compressed air flows downward and acts on the surface of a truncated cone of fiber holder means 9' thereby pushing it together with extraction fiber 3' in the downward direction. This movement is of course linearly guided. For venting chamber 4' a second port 24' is provided in lower probe portion 7'. A corresponding venting line 25' is connected with the ambient atmoshere on the surface.

In its exposed position extraction fiber 3' extends through throughhole 28' in lower end plug 20' and further to the outside of sampling probe 1'. Additionaly, extraction fiber 3' extends in its exposed position through any component of the drilling rod which might be arranged below lower end plug 20'. Accordingly, the downward movement of extraction fiber 3' actuated by the pneumatic source on the surface leads to an immersion of extraction fiber 3' into groundwater and thereby to the desired SPME-process.

After an appropriate equilibration time ,i.e. 30 minutes to several hours, extraction fiber 3' can be retracted from its exposed position to its initial position by means of a retracting means which is in the shown embodiment a retracting or a pull-back spring 26'. Alternatively, the retracting action may also be effected pneumatically by providing suitable pneumatic ports and lines.

Sampling probe 1' has a mounting means, preferably lower end plug 20' and upper end plug 21', for making it capable of being assembled in the head of a drilling rod for drilling the necessary groundwater well. Each end plug 20', 21' has a projection 29', 30' the outer peripheral surface of which has latching edges for engaging corresponding edges in corresponding plugs of the drilling rod. Thereby, sampling probe 1' is tightly secured in the head of the drilling rod.

After the extraction fiber 3' is retracted into chamber 4' the head of the drilling rod is retracted from the groundwater well and extraction fibrer 3' can be again desorbed in a portable gas chromatograph or in a mobile laboratory.

## Claims

1. Sampling probe for the in situ extraction of organic micropollutants from groundwater,
**characterized by**
- a hollow elongated probe housing (2) having a chamber (4) and
- an extraction fiber (3) being coated with an adsorbent material for adsorbing an analyte,
- said extraction fiber (3) being arranged in said chamber (4),
- said probe housing (2) having at least one opening (5) allowing said analyte to enter said chamber (4) in such a manner that said extraction fiber (3) immerses at least partially into said analyte and
- a connector means (6) for connecting a pulling means for lowering and retracting said sampling probe (1) into and from a well.

2. Sampling probe according to claim 1,
**characterized in that**
said probe housing (2) comprises a probe head portion (7) including said chamber (4) and a head holder portion (8) being detachable from each other in order to remove a fiber holder means (9) for holding said extraction fiber (3).

3. Sampling probe according to claim 2,
**characterized in that**
said fiber holder means is a holder plate (9) being clamped between said probe head portion (7) and said head holder portion (8).

4. Sampling probe according to claim 2 or 3,
**characterized in that**
said probe housing (2) comprises an end cap portion (10) being detachable from said head holder portion (8) and having said connector means (6).

5. Sampling probe according to one of the preceding claims,
**characterized in that**
said opening is formed by a plurality of openings for allowing said groundwater to enter said chamber (4), said openings being in the form of radial bores (5) located along the longitudinal axis of said probe housing (2).

6. Sampling probe according to one of claims 1 through 4,
**characterized in that**
said opening is formed by at least one elongated slot extending along the longitudinal axis of said probe housing (2).

7. Sampling probe for the in situ extraction of organic micropollutants from groundwater,
**characterized by**
- a hollow elongated probe housing (2 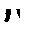 ) having a chamber (4 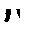 ) and
- an extraction fiber (3 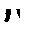 ) being coated with an adsorbent material for adsorbing an analyte,
- said extraction fiber (3') being movably arranged for movement in an axial direction of said probe housing (2') between an initial position in which said extraction fiber (3') is located in said chamber (4') and in an exposed position in which said extraction fiber (3') is exposed to said groundwater outside said chamber (4'), and
- mounting means (20', 21') for mounting said sampling probe (1 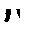 ) in the head of a drilling rod for drilling a well.

8. Sampling probe according to claim 7,
**characterized in that**
said probe housing (2') has a first port (22') for connecting a pressure line (23') connectable to a pneumatic source at the surface in the vicinity of said well.

9. Sampling probe according to claim 8,
**characterized in that**
said probe housing (2') further comprises a second port (24') for connecting a venting line (25') connectable to the ambient atmosphere of said surface.

10. Sampling probe according to claim 8 or 9,
**characterized by**
a fiber holder means (9') for holding said extraction fiber (3') having an actuation surface on which gas delivered from said pneumatic source impinges for actuating said movement.

11. Sampling probe according to claim 10,
**characterized in that**
said actuation surface is a conical surface.

12. Sampling probe according to one of claims 7 through 11
**characterized by**
a retracting means (26') for retracting said extraction fiber (3') from said exposed position to said initial position.

13. Sampling probe according to claim 12
**characterized in that**
said retracting means is a retracting spring (26').

14. Sampling probe according to claim 12 or 13
**characterized in that**
said probe housing (2') comprises a lower probe portion (7') including said chamber (4') and an upper probe portion (8') including said retracting means (26') being detachable from each other.

15. Sampling probe according to claim 14
**characterized in that**
said first port (22') is located in said upper probe portion (8') and said second port (24') is located in said lower probe portion (7').

16. Sampling probe according to one of the preceding claims
**characterized in that**
said extraction fiber is a fused silica fiber (3, 3').
